# EUROPEAN PATENT APPLICATION

(11) **EP 4 578 491 A1**
(43) Date of publication of application: **02.07.2025**
(21) Application number: 24220527.6
(22) Date of filing: 17.12.2024
(51) Int. Cl.: A61N 1/39, A61N 1/04

(54) **SYSTEMS FOR CONVEYING COMPLEX MEDICAL DEVICE OPERATION INSTRUCTIONS**

(30) Priority: 29.12.2023 US 202363616439 P; 10.12.2024 AU 2024278245
(71) Applicant: Stryker Corporation, Portage, MI 49002 (US)
(72) Inventor: DONAGHY, Dymphna Mary, Portage, 49002 (US); ANDERSON, John Houston, Portage, 49002 (US)
(74) Representative: V.O.

(57) **Abstract**

An example method includes illuminating, by a screen, a first treatment icon and a pause icon. The first treatment icon corresponds to a command to administer a first treatment to a subject. The pause icon corresponds to an instruction to refrain from administering a second treatment to the subject. The example method further includes de-illuminating, at the screen, a second treatment icon corresponding to an instruction to administer the second treatment to the subject; determining that a physiological parameter of the subject is indicative of a condition by analyzing the physiological parameter; and in response to determining that the physiological parameter of the subject is indicative of the condition: illuminating, at the screen, the first treatment icon; and de-illuminating the pause icon and the second treatment icon. A treatment is administered to the subject in response to detecting a touch signal.

## Description

### CROSS-REFERENCE TO RELATED APPLICATION

This application claims priority to U.S. Provisional Application No. 63/616,439, which was filed on December 29, 2023 and is incorporated by reference herein in its entirety.

### BACKGROUND

Example medical devices are configured to monitor and/or treat patients using complex techniques. In some cases, these techniques are at least partially automated by the medical devices themselves. However, many medical devices are also manually operated. For instance, electrode pads of an AED are physically connected to a patient by a user before the AED automatically analyzes an electrocardiogram (ECG) of the patient or administers a defibrillation therapy via the electrode pads. Accordingly, it is desirable to design medical devices that coach, or otherwise instruct, users to perform various manual operations

### SUMMARY

According to an aspect is provided an automated external defibrillator (AED), comprising a detection circuit configured to detect an electrical signal indicative of an electrocardiogram (ECG) of a subject. The electrical signal can be received by pads configured to be adhered to the chest of the subject and that can be electrically connected to the detection circuit. The AED comprises a treatment circuit configured to output an electrical shock to the pads. The AED comprises a screen. The screen can comprise a background or a foreground comprising a black color. The screen can comprise a pressure sensor configured to detect a touch signal from a user. The screen can comprise a shock icon corresponding to a shock command. The shock icon can be overlaid on the pressure sensor. The screen can comprise a cardiopulmonary resuscitation (CPR) icon corresponding to an instruction to administer chest compressions. The screen can comprise a hands-off icon corresponding to an instruction to refrain from administering chest compressions. The AED comprises a processor. The processor can be configured to cause the screen to illuminate the shock icon. The processor can be configured to cause the screen to illuminate the hands-off icon. The processor can be configured to cause the screen to blacken the CPR icon. The processor can be configured to in response to causing the screen to illuminate the shock icon, to illuminate the hands-off icon, and to blacken the CPR icon, determine that the ECG is indicative of ventricular fibrillation (VF) by analyzing the ECG. The processor can be configured to in response to determining that the ECG is indicative of VF, cause the screen to illuminate the shock icon, to blacken the hands-off icon, and to blacken the CPR icon. The processor can be configured to in response to causing the screen to illuminate the shock icon, to blacken the hands-off icon, and to blacken the CPR icon, determine that the pressure sensor has detected the touch signal. The processor can be configured to in response to determining that the pressure sensor has detected the touch signal, cause the treatment circuit to output the electrical shock to the pads. The processor can be configured to in response to causing the treatment circuit to output the electrical shock to the pads, cause the screen to blacken the shock icon, to blacken the hands-off icon, and to illuminate the CPR icon.

Optionally, the pressure sensor is a first pressure sensor, and the touch signal is a first touch signal. The screen can comprise a second pressure sensor. The screen can comprise a power icon overlaid on the second pressure sensor. The processor can be further configured to determine that the second pressure sensor has detected a second touch signal. The processor can be further configured to in response to determining that the second pressure sensor has detected the second touch signal, cause the screen to illuminate the power icon, to blacken the shock icon, to blacken the CPR icon, and to blacken the hands-off icon. Optionally, the power icon is disposed between the CPR icon and the hands-off icon.

According to an aspect is provided a medical device. The medical device comprises a sensor configured to detect a physiological parameter of a subject. The medical device comprises a screen. The screen can comprise a pressure sensor configured to detect a touch signal from a user. The screen can comprise a first treatment icon corresponding to a command to administer a first treatment The first treatment icon can be overlaid on the pressure sensor. The screen can comprise a second treatment icon corresponding to an instruction to administer a second treatment. The screen can comprise a pause icon corresponding to an instruction to refrain from administering the second treatment. The medical device comprises a processor. The processor can be configured to cause the screen to illuminate the first treatment icon. The processor can be configured to illuminate the pause icon. The processor can be configured to de-illuminate the second treatment icon. The processor can be configured to determine that the physiological parameter is indicative of a condition by analyzing the physiological parameter. The processor can be configured to in response to determining that the physiological parameter is indicative of the condition, cause the screen to illuminate the first treatment icon, to de-illuminate the pause icon, and to de-illuminate the second treatment icon. The processor can be configured to in response to causing the screen to illuminate the first treatment icon, to de-illuminate the pause icon, and to de-illuminate the second treatment icon, determine that the pressure sensor has detected the touch signal. The processor can be configured to in response to determining that the pressure sensor has detected the touch signal, cause the first treatment to be administered to the subject.

Optionally, de-illuminating the pause icon causes a color of the pause icon to match a color of a background or a foreground of the screen, and/or wherein de-illuminating the second treatment icon causes a color of the second treatment icon to match the color of the background or the foreground of the screen.

Optionally, the medical device further comprises a treatment circuit configured to administer the first treatment. The first treatment can comprise an electrical shock or pacing pulses.

Optionally, the second treatment comprises chest compressions.

Optionally, the medical device is an automated external defibrillator (AED). The sensor can comprises a detection circuit configured to detect an electrical signal indicative of an electrocardiogram (ECG) of a subject. The electrical signal can be received by pads configured to be adhered to the chest of the subject and that can be electrically connected to the detection circuit. The treatment circuit can be configured to output an electrical shock to the pads. The screen can comprise a background or a foreground comprising a black color. The first treatment icon can be a shock icon corresponding to a shock command. The shock icon can be overlaid on the pressure sensor. The second treatment icon can be a cardiopulmonary resuscitation (CPR) icon corresponding to an instruction to administer chest compressions. The pause icon can be a hands-off icon corresponding to an instruction to refrain from administering chest compressions. The processor can be configured to cause the screen to illuminate the shock icon, to illuminate the hands-off icon, and to blacken the CPR icon. The processor can be configured to in response to causing the screen to illuminate the shock icon, to illuminate the hands-off icon, and to blacken the CPR icon, determine that the ECG is indicative of ventricular fibrillation (VF) by analyzing the ECG. The processor can be configured to in response to determining that the ECG is indicative of VF, cause the screen to illuminate the shock icon, to blacken the hands-off icon, and to blacken the CPR icon. The processor can be configured to in response to causing the screen to illuminate the shock icon, to blacken the hands-off icon, and to blacken the CPR icon, determine that the pressure sensor has detected the touch signal. The processor can be configured to in response to determining that the pressure sensor has detected the touch signal, cause the treatment circuit to output the electrical shock to the pads. The processor can be configured to in response to causing the treatment circuit to output the electrical shock to the pads, cause the screen to blacken the shock icon, to blacken the hands-off icon, and to illuminate the CPR icon.

Optionally, the condition comprises VF, pulseless ventricular tachycardia (VT), or bradycardia.

Optionally, the medical device further comprises the pressure sensor being a first pressure sensor. The screen can further comprise a second pressure sensor. The screen can further comprise a power icon overlaid on the second pressure sensor. The processor can further be configured to, in response to determining that the physiological parameter is indicative of the condition, cause the screen to de-illuminate the power icon.

Optionally, the processor is further configured to determine that the second pressure sensor has detected a second touch signal; and in response to determining that the second pressure sensor has detected the second touch signal, cause the screen to illuminate the power icon, to blacken the shock icon, to blacken the CPR icon, and to blacken the hands-off icon.

Optionally, the power icon is disposed between the CPR icon and the hands-off icon.

Optionally, the touch signal is a first touch signal, and the medical device can be configured to power on in response to the first pressure sensor or the second pressure sensor detecting a second touch signal.

Optionally, causing the screen to illuminate the first treatment icon, to de-illuminate the pause icon, and to de-illuminate the second treatment icon comprises exclusively illuminating the first treatment icon and hiding other icons comprising the pause icon and the second treatment icon. The other icons optionally further comprise a setup icon corresponding to an instruction to connect the sensor to the subject, a language selection icon, a pediatric mode icon, and a power icon.

According to an aspect is provided a method, comprising illuminating, at a screen, a first treatment icon and a pause icon, the first treatment icon corresponding to a command to administer a first treatment to a subject, the pause icon corresponding to an instruction to refrain from administering a second treatment to the subject. The method comprises de-illuminating, at the screen, a second treatment icon corresponding to an instruction to administer the second treatment to the subject. The method can comprise determining that a physiological parameter of the subject is indicative of a condition by analyzing the physiological parameter. The method can comprise in response to determining that the physiological parameter of the subject is indicative of the condition illuminating, at the screen, the first treatment icon, and de-illuminating the pause icon and the second treatment icon. The method can comprise detecting, by a pressure sensor e.g. overlaid by the first treatment icon, a touch signal. The method can comprise in response to detecting the touch signal, generating a command to cause administration of the first treatment to the subject. The instruction can be configured to automatically start administration of the first treatment to the subject. The method can be a computer implemented method, such as performed by a processor, e.g. the processor of the medical device or AED described herein. The method can comprise in response to detecting the touch signal, the processor causing administration of the first treatment to the subject.

Optionally, de-illuminating the pause icon causes a color of the pause icon to match a color of a background or a foreground of the screen. Optionally, de-illuminating the second treatment icon causes a color of the second treatment icon to match the color of the background or the foreground of the screen.

Optionally, the first treatment comprises an electrical shock or pacing pulses. Optionally, the second treatment comprises chest compressions. Optionally, the second treatment comprises assisted ventilation.

Optionally, the condition comprises VF, pulseless ventricular tachycardia (VT), or bradycardia.

Optionally, the touch signal is a first touch signal, and the method further comprises detecting, by the pressure sensor, e.g. overlaid by the first treatment icon, a second touch signal. The method can comprise in response to detecting the second touch signal, powering on a medical device comprising the screen.

Optionally, illuminating the first treatment icon and de-illuminating the pause icon and the second treatment icon comprises exclusively illuminating the first treatment icon and hiding other icons comprising the pause icon and the second treatment icon.

It will be appreciated that any of the aspects, features and options described herein can be combined. It will particularly be appreciated that any of the aspects, features and options described in view of the medical device apply equally to the AED and methods, and vice versa.

### BRIEF DESCRIPTION OF THE DRAWINGS

FIG. 1 illustrates an example environment in which a medical device provides enhanced instructions to a user for operating the medical device in order to monitor and treat a condition of a patient.
FIG. 2 is an illustration of an example AED with an enhanced user interface.
FIGS. 3A to 3E illustrate various modes of an AED with an enhanced user interface.
FIG. 4 illustrates an example process for presenting an enhanced user interface for guiding a user in operating a medical device.
FIG. 5 illustrates another example process for presenting an enhanced user interface for guiding a user in operating a medical device.
FIG. 6 illustrates an example of an external defibrillator configured to perform various functions described herein.
FIG. 7 illustrates an example of a screen of a medical device, which can implement various enhanced user interfaces described herein.

### DETAILED DESCRIPTION

Various implementations described herein relate to enhanced graphical user interfaces (GUIs) enabling medical devices to be operated by untrained users. For instance, implementations of the present disclosure enable bystanders to operate an automated external defibrillator (AED), or other emergency medical device, without prior training.

Certain medical devices are operated by trained users, such as clinicians, emergency medical technicians, and the like. Some medical devices, however, are designed to be operated by users who lack specialized training. Many of these medical devices, for instance, are designed to treat medical emergencies in non-clinical settings. For instance, AEDs are often stored in non-clinical, public areas so that they are near the locations of individuals who experience sudden cardiac arrest or other ailments. Because they are stored in non-clinical settings, it is unlikely that trained caregivers are available to operate them. However, it can be challenging to encourage untrained users to accurately, efficiently, and rapidly operate medical devices, such as in high-stress environments to treat individuals experiencing acute medical emergencies.

In particular cases, medical devices with multiple functions (e.g., monitoring and treatment capabilities) may be designed with complex user interfaces. However, untrained users in stressful operating conditions can get distracted and flustered by complex user interfaces, which can delay care to patients experiencing medical emergencies. Studies have demonstrated that even turning on a medical device can be challenging to untrained users who are eager to provide assistance to incapacitated patients.

Various implementations of the present disclosure address these and other problems by providing enhanced user interfaces that instruct untrained users how to operate medical devices. An example medical device includes a display that selectively activates and deactivates various icons that guide the timing and performance of manual operations of the medical device during an emergency event. In particular cases, icons corresponding to instructions that are irrelevant to a particular stage of the operation of the device are actively hidden from view of the user during the particular stage. For example, the display visually presents a background color of the display in an area corresponding to a hidden icon. In some cases, an example medical device includes multiple icons corresponding to multiple input devices that, when activated by a user, trigger the same or similar actions. For instance, the medical device may have multiple icons, wherein the medical device turns on when any one of the multiple icons is selected by a user. Various techniques described herein can greatly simplify operation of medical devices for untrained users, which can decrease delays until patients experiencing medical emergencies can be monitored and/or treated.

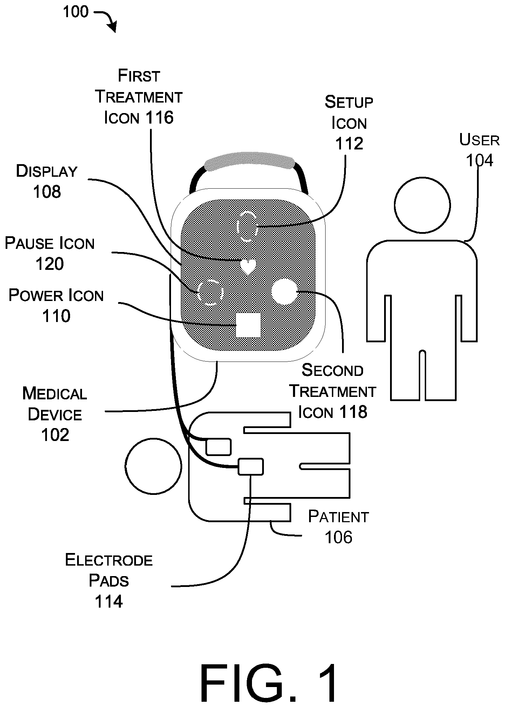

FIG. 1 illustrates an example environment 100 in which a medical device 102 provides enhanced instructions to a user 104 for operating the medical device 102 in order to monitor and treat a condition of a patient 106. In various implementations, the example environment 100 includes a non-clinical setting. For example, the patient 106 may be experiencing a medical emergency outside of a clinical setting, such as a hospital. In particular instances, the patient 106 may have suddenly collapsed in a public area, such as a school, airport terminal, or office building.

In some cases, the patient 106 is experiencing sudden cardiac arrest. For example, the patient 106 may have a cardiac arrhythmia that prevents the heart of the patient 106 from effectively pumping blood through the body of the patient 106. As a result of the lack of oxygenated blood flow through the body of the patient 106, various tissues in the body of the patient 106 may eventually experience a hypoxic injury. In particular cases, a sustained lack of blood flow can cause damage to the brain and other vital organs of the patient 106.

To avoid significant and permanent damage to the patient 106, the medical device 102 may be configured to administer a treatment that monitors and/or treats the medical emergency of the patient 106. According to some examples, the medical device 102 is stored in the non-clinical setting. Thus, the medical device 102 may be used to provide rapid care to the patient 106 before the patient 106 is transported to a clinical setting, such as a hospital.

In order to administer care to the patient 106 in the non-clinical setting, the medical device 102 is configured to be used by bystanders with limited to no medical training. The user 104 operating the medical device 102 is an untrained user, in various examples. For example, the user 104 may be an individual in the non-clinical setting who happened to be present when the patient 106 began to experience the medical emergency. In some cases, the user 104 has removed the medical device 102 from a cabinet or other storage mechanism disposed in the non-clinical setting, and has brought the medical device 102 to the side of the patient 106. According to some examples, the medical device 102 is portable.

The medical device 102 provides instructions to the user 104 for operating the medical device. Accordingly, the user 104 may utilize the medical device 102 to administer a complex treatment to the patient 106 despite their lack of specialized medical care. In some cases, a particular order and/or timing of instructions are presented by the medical device 102. The order and/or timing of instructions, for instance, may be triggered based on a detected condition of the patient 106. In some cases, the order and/or timing of the instructions is triggered based on an operation of the medical device 102 by the user. According to various examples, the medical device 102 outputs audio instructions to the user 104 that guide the use of the medical device 102.

At least some of the instructions are visually presented on a display 108. The display 108, in various implementations, may have a solid background or foreground color. For instance, the display 108 may have a black background or foreground color. In some examples, the display 108 is a touchscreen. In various examples, the display 108 includes one or more touch sensors integrated with a display screen. The touch sensors, for instance, include pressure sensors, capacitive sensors (e.g., capacitive membrane sensors), resistive sensors, or a combination thereof. In some examples, the touch sensors within the display 108 are configured to detect a touch of the user 104 on the display 108.

For example, in a first area of the display 108, the medical device 102 visually presents a power icon 110. The first area of the display overlaps one or more first touch sensors. According to some examples, the medical device 102 is configured to power on when the first touch sensor(s) detect a touch signal from the user 104. Once powered on, in various examples, the medical device 102 initiates a functional protocol that instructs the user 104 on how to setup the medical device 102 to monitor and/or treat the patient 106.

In some implementations, the power icon 110 is printed on a surface of the display 108 with opaque ink, such that it is visible at all times. When the medical device 102 is powered off, in some cases, the power icon 110 is illuminated. For instance, one or more light sources (e.g., LEDs) that are integrated with the power icon 110 are illuminated and/or flash periodically when the medical device 102 Is powered off. In some cases, the light source(s) flash a first color when the medical device 102 is ready for use (e.g., a battery of the medical device 102 has greater than a predetermined charge level) and may flash a second color when the medical device 102 is not ready for use (e.g., the battery has less than the predetermined charge level). The light source(s), for instance, may flash periodically when the medical device 102 is powered off.

In some cases, the power icon 110 is only visible when it is illuminated by the display 108. In some implementations, the power icon 110 is de-illuminated when the medical device 102 is powered on. In some cases, the power icon 110 is illuminated when the medical device 102 is powered off. For example, the medical device 102 may be powered off when the first touch sensor(s) detect a touch signal from the user 104. In some examples, the medical device 102 powers off when the power icon 110 is selected by the user 104.

In a second area of the display 108, the medical device 102 visually presents setup icon 112. In various examples, the setup icon 112 visually instructs the user 104 to connect the medical device 102 to the patient 106. For example, the setup icon 112 instructs the user 104 to place electrode pads 114 on the patient 106. In various cases, the electrode pads 114 include electrodes configured to receive an electrical signal indicative of an activity of the heart of the patient 106. Further, the electrodes in the electrode pads 114 may be configured to output an electrical signal (e.g., a defibrillating electrical shock, pacing pulses, etc.) to the heart of the patient 106. In various implementations, the electrode pads 114 are configured to be applied externally to the patient 106. For instance, the electrode pads 114 are configured to be adhered to the skin of the patient 106. According to some examples, a biocompatible and electrically conductive adhesive is disposed on a surface of the electrode pads 114, enabling a secure and adequate electrical connection between the skin of the patient 106 and the electrodes of the electrode pads 114. In some examples, the setup icon 112 visually shows the user 104 a preferred positioning of the electrode pads 114 on the chest of the patient 106.

In a third area of the display 108, the medical device 102 presents a first treatment icon 116. The third area of the display 108, for instance, overlaps one or more second touch sensors. In various cases, the medical device 102 is configured to prepare for and/or output a first treatment when the second touch sensor(s) of the display 108 detects a touch signal from the user 104.

According to some examples, the medical device 102 prepares for the first treatment by charging at least one capacitor in the medical device 102. Further, the medical device 102 may administer the first treatment by discharging the capacitor(s) via the electrode pads 114 in the form of one or more electrical shocks. In some cases, the first treatment defibrillates the patient 106. In some examples, the first treatment includes administering pacing pulses to the patient 106.

In a fourth area of the display 108, the medical device 102 presents a second treatment icon 118. In various examples, the second treatment icon 118 instructs the user 104 to administer a second treatment. According to various examples, the second treatment is a manual treatment. For example, the second treatment may include the user 104 manually administering chest compressions to the patient 106. In some examples, the second treatment includes the user 104 administering assisted ventilation to the patient 106.

In a fifth area of the display 108, the medical device 102 presents a pause icon 120. The pause icon 120, for instance, visually instructs the user 104 to at least temporarily refrain from administering the second treatment to the patient 106. For instance, the pause icon 120 may be a "hands-off" icon that instructs the user 104 to remove their hands from the surface of the patient 106. In various cases, the medical device 102 is configured to output an electrical signal (e.g., an electrical shock or pacing pulses) to the patient 106 when the pause icon 120 is activated. Therefore, the pause icon 120 may prevent the user 104 from receiving the electrical signal output by the medical device 102.

In various implementations, the medical device 102 selectively activates (e.g., displays) and deactivates (e.g., hides) various icons on the display 108. In particular cases, the medical device 102 deactivates at least some of icons at particular times in order to avoid misleading the user 104 into improperly operating the medical device 102. According to some examples, the medical device 102 deactivates at least some of the icons by de-illuminating them on the display 108. In particular examples, the medical device 102 hides at least some of the icons by displaying the background color or foreground color at the locations of the display 108 corresponding to the icons being hidden.

In various cases, icons that are deactivated by the display 108 of the medical device are imperceptible to the user 104. In some examples, the display 108 includes physical pixels that are configured to emit less than a threshold amount of light when they correspond to a deactivated icon. For instance, in some cases, the display 108 includes organic light-emitting diode (OLED) pixels that refrain from outputting light when they are deactivated. In some cases, the icons of the display 108 include backlit icons that are selectively illuminated when activated. In some cases, the display 108 includes a light valve layer that selectively opens, thereby letting light from a light-emitting layer of the display 108 to illuminate the icons. In some examples, the display 108 includes active light-emitting elements that are selectively activated in order to cause the illumination of the icons. However, when the backlit icons are not illuminated, the user 104 may be unable to perceive the de-illuminated backlit icons. For instance, the display 108 may have a coating that prevents the de-illuminated backlit icons from being perceived by the user 104 via reflection. In some cases, the coating is n antireflective coating, such as a thin-film structure including alternating layers of materials with different refractive indices. Thus, the hidden icons are distinct from more conventional de-illuminated backlit icons, which can be seen in a de-illuminated form.

In particular cases, the medical device 102 operates in various instruction modes that each selectively present one or more of the icons. In a first mode, in which the medical device 102 is stored (such as in a cabinet), the medical device 102 may present one or more icons that enable the user 104 to activate the medical device 102. In particular examples, the power icon 110 is activated in the first mode. Further, in some examples, the first treatment icon 116 is also displayed in the first mode. Other icons, such as the setup icon 112, the second treatment icon 118, and the pause icon 120 are hidden in the first mode. Accordingly, the display 108 visually presents a limited and simplified collection of icons that enable the user 104 to activate the medical device 102 without significant distractions.

The medical device 102 initiates a second mode in response to first touch sensor(s) and/or the second touch sensor(s) of the display 108 detecting a touch signal of the user 104. That is, the user 104 may activate the medical device 102 by touching or pressing the power icon 110 or the first treatment icon 116.

In the second mode, in which the medical device 102 is initially activated, the medical device 102 may present one or more icons that enable the user 104 to prepare the medical device 102 and/or patient 106 for monitoring and/or treatment. In various cases, the medical device 102 presents the setup icon 112. To avoid confusing the user 104, the medical device may specifically hide one or more of the icons, such as the power icon 110, the second treatment icon 118, or the pause icon 120. In some cases, the medical device 102 presents the first treatment icon 116. In some examples, the medical device 102 hides the first treatment icon 116 in the second mode.

The medical device 102 may transition from the second mode to a third mode or a fourth mode in response to detecting an electrical signal from the patient 106. For example, the medical device 102 may initiate the third mode in response to detecting that the electrode pads 114 are positioned on the chest of the patient 106. In some examples, the medical device 102 determines that the electrode pads 114 are positioned by detecting an ECG of the patient 106 via the electrode pads 114. In some cases, the medical device 102 transitions from the second mode to the third mode based on an analysis of the ECG. For instance, the medical device 102 may transition from the second mode to the third mode upon determining that the ECG is indicative of an arrhythmia that lacks QRS complexes indicative of a normal sinus rhythm.

According to some cases, the medical device 102 may instruct the user 104 to administer the second treatment to the patient 106 in the third mode. For example, the medical device 102 may activate the second treatment icon 118. In various cases, the medical device 102 may hide the power icon 110, the setup icon 112, the first treatment icon 116, the pause icon 120, or any combination thereof, in the third mode. By hiding various icons, the medical device 102 may prevent the user 104 from efficiently perceiving the instruction to administer the second treatment. Accordingly, the third mode of the medical device 102 may decrease the time until the user 104 begins administering the chest compressions to the patient 106.

In various implementations, the medical device 102 transitions from the third mode to the fourth mode when a predetermined time has expired. For example, the medical device 102 may automatically transition from the third mode to the fourth mode when the medical device 102 has been in the third mode for a predetermined time period (e.g., two minutes).

In various cases, the medical device 102 instructs the user to refrain from administering the second treatment to the patient 106 in the fourth mode. For example, the medical device 102 may activate the pause icon 120. However, in the fourth mode, the medical device 102 may deactivate at least one of the power icon 110, the setup icon 112, the first treatment icon 116, the second treatment icon 118, or any combination thereof. Accordingly, the medical device 102 may prevent the untrained user 104 from administering the second treatment while the medical device 102 is in the fourth mode.

According to some cases, the medical device 102 analyzes the physiological parameter when the medical device 102 is in the fourth mode. For instance, the second treatment may induce an artifact (e.g., a chest compression artifact, a ventilation artifact, or some other type of motion artifact) in data indicative of the physiological parameter. By instructing the user 104 to at least temporarily refrain from administering the second treatment, the medical device 102 may improve the signal quality of the physiological parameter of the patient 106 detected by the medical device 102. In some implementations, the medical device 102 determines whether the physiological parameter is indicative of a condition that is treatable by the first treatment while the medical device 102 is in the fourth mode. For example, if the first treatment is an electrical shock, the medical device 102 may determine whether the ECG of the patient 106 is indicative of a shockable arrhythmia, such as VF or pulseless VT. In cases where the first treatment includes pacing pulses, the medical device 102 may determine whether the ECG, heart rate, or pulse rate of the patient 106 is indicative of bradycardia. In some cases, the medical device 102 prepares to administer the first treatment upon determining that the physiological parameter is indicative of the condition. For example, the medical device 102 may begin charging a capacitor upon determining that the physiological parameter is indicative of the condition.

In various cases, the medical device 102 transitions from the fourth mode to a fifth mode if the medical device 102 determines that the physiological parameter is indicative of the condition that is treatable by the first treatment. However, in some cases, the medical device 102 transitions from the fourth mode back to the third mode if the medical device 102 is unable to determine that the physiological parameter is indicative of the condition, the medical device 102 has been in the fourth mode for greater than a threshold time period (e.g., 30 seconds), the medical device 102 has not completed its preparation for administering the first treatment (e.g., the capacitor has not been fully charged to a predetermined amount), or any combination thereof. If the medical device 102 transitions back to the third mode due to the medical device 102 not yet completing its preparation for administering the first treatment, the medical device 102 may continue its preparations for the first treatment while temporarily operating in the third mode and may transition from the third mode to the fifth mode upon completing the preparation for administering the first treatment.

In various examples, the medical device 102 instructs the user 104 to trigger administration of the first treatment when the medical device 102 is in the fifth mode. In various cases, if the patient 106 has the condition that is treatable by the first treatment, the patient 106 may be particularly vulnerable to delays in care. Therefore, in various implementations, the medical device 102 may present a specific combination of icons that are designed to quickly convey to the user 104 about the need to administer the first treatment and instructions for triggering administration of the first treatment. In various cases, the first treatment is administered, at least in part, by the medical device 102. The medical device 102, for instance, triggers the administration of the first treatment is in response to an input signal that the medical device 102 detects from the user 104.

The medical device 102 may activate the first treatment icon 116 in the fifth mode. In some cases, the medical device 102 emphasizes the first treatment icon 116. For example, the medical device 102 may cause the first treatment icon 116 to brighten and/or blink in the fifth mode. According to various implementations, the medical device 102 specifically hides all other icons besides the first treatment icon 116. For instance, the medical device 102 hides the power icon 110, the setup icon 112, the second treatment icon 118, and the pause icon 120 in the fifth mode. Various techniques described herein may prevent the user 104 from misinterpreting the urgent recommendation to administer the first treatment in the fifth mode.

In various implementations, the medical device 102 enters a sixth mode in response to the touch sensor(s) overlapping the first treatment icon 116 detecting an input signal (e.g., a touch) of the user 104. During the sixth mode, in various cases, the medical device 102 instructs the user 104 to refrain from touching the patient 106. For instance, the medical device 102 may activate the pause icon 120. In some implementations, the medical device 102 outputs other signals to the user 104, such as an audio command, instructing the user 104 to refrain from touching the patient 106. In various cases, the medical device 102 outputs the first treatment to the patient 106. For example, the medical device 102 outputs at least one electrical shock to the electrode pads 114.

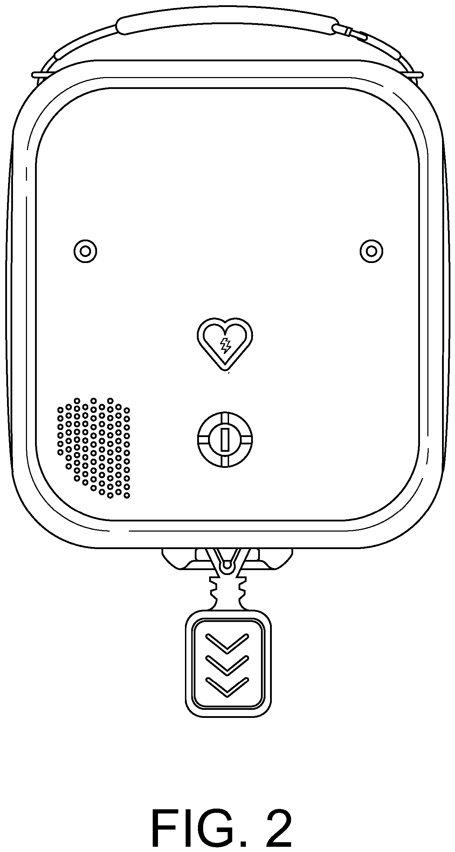

FIG. 2 is an illustration of an example AED 200 with an enhanced user interface. For example, the AED 200 includes a defibrillation icon 202 and a power icon 204. In some implementations, the AED 200 corresponds to the medical device 102; the defibrillation icon 202 corresponds to the first treatment icon 116; and the power icon 204 corresponds to the power icon 110. In various implementations, FIG. 2 illustrates the AED 200 when the AED 200 is in long-term storage, such as in a cabinet. For instance, the AED 200 may be activated if a user touches the defibrillation icon 202 or the power icon 204. That is, a user may activate the AED 200 without touching the power icon 204, even though the power icon 204 is activated when the AED 200 is in storage.

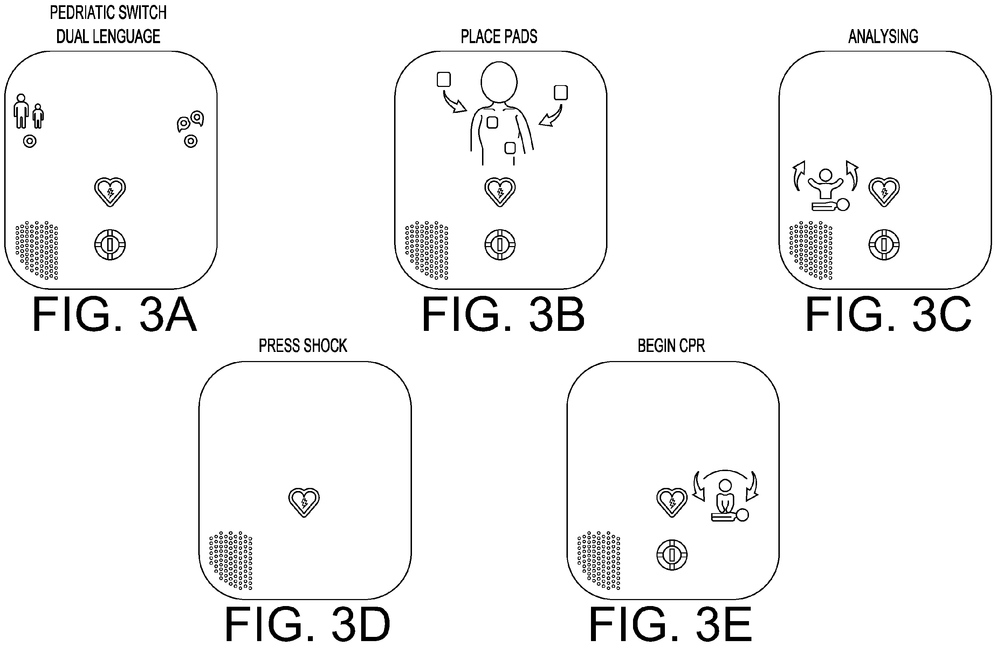

FIGS. 3A to 3E illustrate various modes of an AED with an enhanced user interface. In various implementations, the AED illustrated in FIGS. 3A to 3E corresponds to the medical device 102 described above with reference to FIG. 1.

FIG. 3A illustrates a mode in which the AED has been activated. During this mode, a pediatric switch icon and a language switch icon are activated on a display of the AED. For example, the pediatric switch icon may enable the AED to monitor and/or treat a pediatric patient (e.g., an individual who is less than a threshold age) or an adult patient (a non-pediatric patient). In various cases, the language switch icon enables the AED to select a language of instructions output by the AED. For example, after language selection, the AED may output audible commands in the selected language. In various cases, the AED selects a pediatric mode and/or language based on touch sensors overlapping the pediatric switch icon and the language switch icon detecting one or more touch signals from a user. In the mode of FIG. 3A, the AED also activates a power icon and a defibrillation icon. However, in some implementations, the power icon is deactivated when the AED is powered on and is thus deactivated in this mode.

FIG. 3B illustrates a mode in which the AED instructs the user to connect the AED to a patient. In this mode, the AED activates an icon instructing the user to apply electrode pads to specific regions of the physiology of the patient. In this mode, the power icon and the defibrillation icon remain activated. In some implementations, the power icon is deactivated when the AED is powered on, and is thus deactivated in this mode.

FIG. 3C illustrates a mode in which the AED instructs the user to refrain from administering chest compressions to the patient. In this mode, the AED activates a pause icon that illustrates hands away from a patient. The AED further keeps the power icon and the defibrillation icon in this mode. During this mode, the AED may analyze an ECG of the patient based on an electrical signal detected from the electrode pads. In some implementations, the power icon is deactivated when the AED is powered on, and is thus deactivated in this mode.

FIG. 3D illustrates a mode in which the AED instructs the user to initiate administration of a defibrillation therapy. In this mode, the AED has deactivated all icons besides the defibrillation icon. In some cases, the defibrillation icon is blinking in this mode. Upon one or more touch sensors overlapping the defibrillation icon detecting a touch signal from a user, the AED may output an electrical shock to the electrode pads. The power icon is deactivated in this mode.

FIG. 3E illustrates a mode in which the AED instructs the user to initiate chest compressions. During this mode, the AED activates a chest compression icon. The defibrillation icon and the power icon remain activated in this mode. In some implementations, the power icon is deactivated when the AED is powered on, and is thus deactivated in this mode.

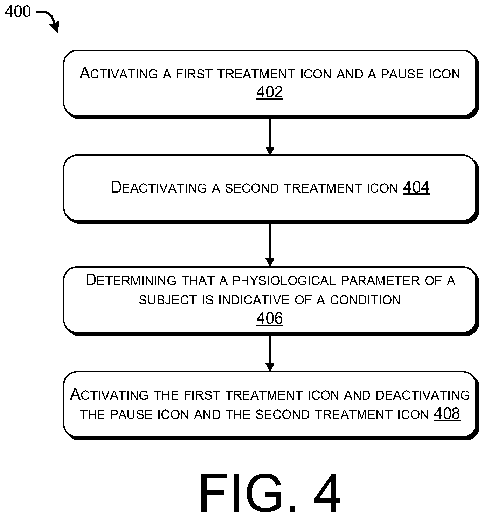

FIG. 4 illustrates an example process 400 for presenting an enhanced user interface for guiding a user in operating a medical device. The process 400 may be performed by an entity including a medical device (e.g., the medical device 102), a defibrillator (e.g., the AED of FIG. 2 and/or the AED of FIGS. 3A to 3E), a ventilator, a mechanical chest compression device, at least one processor, a computing device, a display (e.g., the display 108), or any combination thereof.

At 402, the entity activates a first treatment icon and a pause icon. In various cases, the first treatment icon includes a command, instruction, or suggestion to administer a first treatment to a subject. In various cases, the first treatment corresponds to a therapy that is administered by a medical device, such as a medical device included in the entity. Examples of the first treatment include, for instance, pacing pulses, an electrical shock (e.g., a defibrillation treatment), mechanical chest compressions, or assisted ventilation. The pause icon, for instance, includes a command, instruction, or suggestion to refrain from administering a second treatment to the subject. The second treatment, for example, corresponds to a therapy that is administered by a rescuer. For example, the second treatment may include manual chest compressions, manual assisted ventilation, administration of a medication, or any combination thereof.

According to various examples, the entity activates the first treatment icon and the pause icon by illuminating the first treatment icon and the pause icon on a display. In some cases, the display is a screen integrated with one or more touch sensors. For example, the first treatment icon, when activated, is overlaid on a touch sensor (e.g., a pressure sensor and/or a capacitive sensor) in the screen. In some examples, the first treatment icon and the pause icon are backlit shapes on the display. According to some implementations, prior to being activated, the first treatment icon and the pause icon are not discernible on the display. For example, areas corresponding to the first treatment icon and the pause icon display a background or foreground color of the display prior when the first treatment icon and the pause icon are not activated.

At 404, the entity deactivates a second treatment icon. The second treatment icon, for example, includes a command, instruction, or suggestion to administer the second treatment to the subject. When the second treatment icon is deactivated, the second treatment icon is not discernible on the display. For example, an area corresponding to the second treatment icon on the display matches a background or foreground color of the display when the second treatment icon is deactivated. If the background or foreground color is black, the entity may blacken the area corresponding to the second treatment icon. In some cases, a backlight corresponding to the second treatment icon refrains from outputting light when the second treatment icon is deactivated.

At 406, the entity determines that a physiological parameter of the subject is indicative of a condition. In various cases, the entity includes a sensor that is configured to detect the physiological parameter. Examples of the physiological parameter include, for instance, an ECG, a heart rate, a pulse rate, a blood velocity through at least one blood vessel of the subject, a capnograph, an end-tidal carbon dioxide, an end-tidal oxygen, a blood oxygenation (e.g., a pulse oxygenation value), a blood pressure, a temperature, a respiration rate, or any combination thereof. The entity, for example, generates data indicative of the physiological parameter and identifies the condition by analyzing the data. The condition, for example, may include VF, pulseless VT, bradycardia, or any combination thereof. In some cases, the condition is detected by determining that the physiological parameter is outside of a predetermined range. For example, the entity determines that the physiological parameter has remained above a first threshold, or has remained below a second threshold, for greater than a threshold period of time (e.g., 30 seconds, 1 minute, or the like). According to various cases, the condition is treatable by the first treatment.

At 408, the entity activates the first treatment icon and deactivates the pause icon and the second treatment icon. For instance, the entity illuminates the first treatment icon and de-illuminates the pause icon and the second icon. In some implementations, at 408, the only active icon on the display of the entity is the first treatment icon. For example, other icons that are activated on the display at other times (e.g., a setup icon, a language selection icon, a pediatric mode icon, a power icon, and the like) are deactivated at 408. According to some examples, the display exclusively activates the first treatment icon on the display. Areas corresponding to other icons, for instance, display the background or foreground color at 408. Accordingly, a user is prevented from being distracted by other icons, instructions, or other shapes when the first treatment icon is activated at 408. According to some examples, the entity further causes administration of the first treatment in response to detecting a touch at the pressure sensor overlapping the first treatment icon on the display. Thus, the process 400 can be used to reduce the time until the

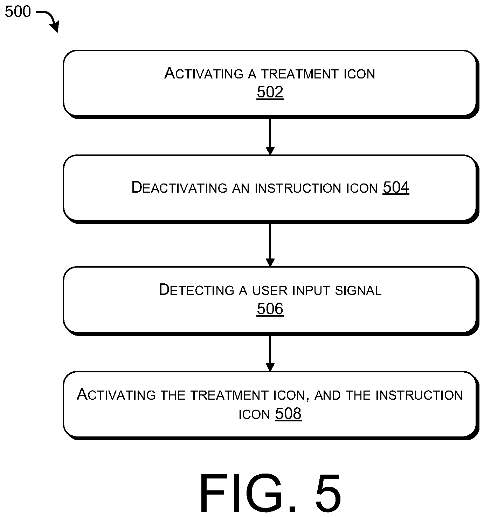

FIG. 5 illustrates another example process 500 for presenting an enhanced user interface for guiding a user in operating a medical device. The process 500 may be performed by an entity including a medical device (e.g., the medical device 102), a defibrillator (e.g., the AED of FIG. 2 and/or the AED of FIGS. 3A to 3E), a ventilator, a mechanical chest compression device, at least one processor, a computing device, a display (e.g., the display 108), or any combination thereof.

At 502, the entity activates a treatment icon. In various cases, the treatment icon includes a command, instruction, or suggestion to administer a first treatment to a subject. In various cases, the first treatment corresponds to a therapy that is administered by a medical device, such as a medical device included in the entity. Examples of the treatment include, for instance, pacing pulses, an electrical shock (e.g., a defibrillation treatment), mechanical chest compressions, or assisted ventilation. In various cases, the treatment icon is located in a center of the display. Optionally, the entity activates a power icon at 502.

In various implementations, the treatment icon and the power icon are visual icons output on a display of the medical device. In some cases, the display is a screen integrated with one or more touch sensors. The treatment icon, for instance, overlaps a first touch sensor. The power icon, for instance, overlaps a second touch sensor. When activated, the treatment icon and the power icon may be illuminated, or otherwise visible, on the display. In various cases, the entity performs 502 while the medical device is in long-term storage. For example, the medical device is in a power-saving state when the entity performs 502. In some examples, a battery of the medical device is disconnected from a physiological sensor of the medical device when the entity performs 502.

At 504, the entity deactivates an instruction icon. According to various implementations, the instruction icon is de-illuminated on the display at 504. For example, an area of the display corresponding to the instruction icon may be a background or foreground color of the display. If the background or foreground color is black, the entity may blacken the area of the display corresponding to the instruction icon. In various cases, the instruction icon is a backlit icon, and a light configured to illuminate the instruction icon is turned off at 504. The instruction icon, in various cases, includes an instruction to connect the medical device to a subject, to administer another treatment to the subject, or to at least temporarily refrain from administering the other treatment to the subject. For instance, the instruction icon may include an instruction to connect electrode pads to the chest of the subject.

At 506, the entity detects a user input signal. According to various implementations, the user input signal is detected by the first touch sensor overlapping the treatment icon or by the second touch sensor overlapping the power icon. In various implementations, the entity transitions from a storage mode to an active mode when the entity detects the user input signal. A user may therefore turn on the medical device by activating the treatment icon or the power icon.

At 508, the entity activates the treatment icon and the instruction icon. According to various cases, the entity illuminates the power icon, the treatment icon, and the instruction icon. In some implementations, the entity subsequently monitors and/or administers a treatment to a subject. In some examples, the power icon is deactivated at 508.

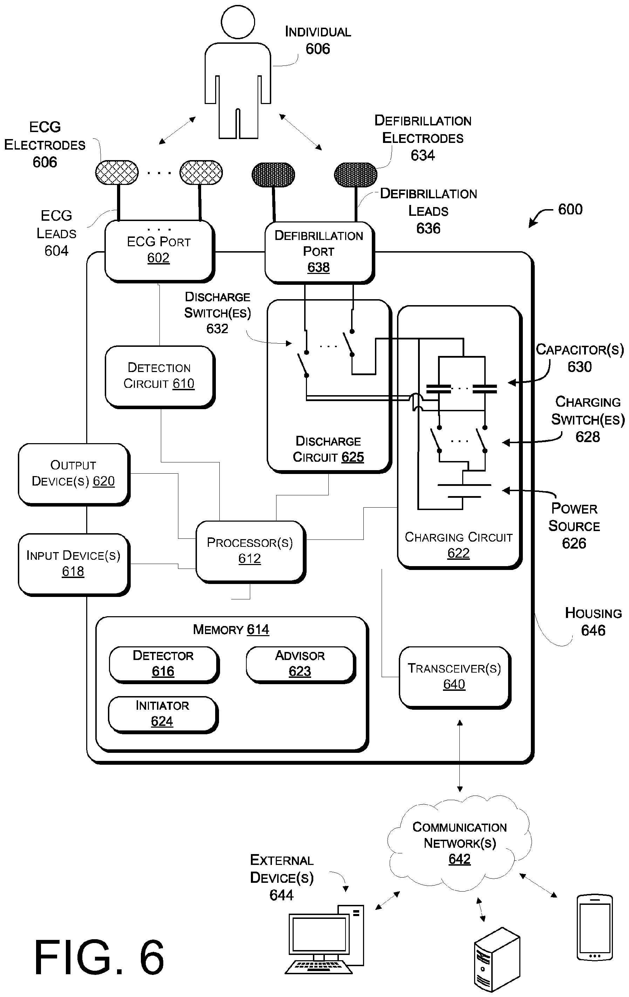

FIG. 6 illustrates an example of an external defibrillator 600 configured to perform various functions described herein. For example, the external defibrillator 600 is the medical device 102 described above with reference to FIG. 1.

The external defibrillator 600 includes an electrocardiogram (ECG) port 602 connected to multiple ECG leads 604. In some cases, the ECG leads 604 are removeable from the ECG port 602. For instance, the ECG leads 604 are plugged into the ECG port 602. The ECG leads 604 are connected to ECG electrodes 606, respectively. In various implementations, the ECG electrodes 606 are disposed on different locations on an individual 608. A detection circuit 610 is configured to detect relative voltages between the ECG electrodes 606. These voltages are indicative of the electrical activity of the heart of the individual 608.

In various implementations, the ECG electrodes 606 are in contact with the different locations on the skin of the individual 608. In some examples, a first one of the ECG electrodes 606 is placed on the skin between the heart and right arm of the individual 608, a second one of the ECG electrodes 606 is placed on the skin between the heart and left arm of the individual 608, and a third one of the ECG electrodes 606 is placed on the skin between the heart and a leg (either the left leg or the right leg) of the individual 608. In these examples, the detection circuit 610 is configured to measure the relative voltages between the first, second, and third ECG electrodes 606. Respective pairings of the ECG electrodes 606 are referred to as "leads," and the voltages between the pairs of ECG electrodes 606 are known as "lead voltages." In some examples, more than three ECG electrodes 606 are included, such that 5-lead or 12-lead ECG signals are detected by the detection circuit 610.

The detection circuit 610 includes at least one analog circuit, at least one digital circuit, or a combination thereof. The detection circuit 610 receives the analog electrical signals from the ECG electrodes 606, via the ECG port 602 and the ECG leads 604. In some cases, the detection circuit 610 includes one or more analog filters configured to filter noise and/or artifact from the electrical signals. The detection circuit 610 includes an analog-to-digital (ADC) in various examples. The detection circuit 610 generates a digital signal indicative of the analog electrical signals from the ECG electrodes 606. This digital signal can be referred to as an "ECG signal" or an "ECG."

In some cases, the detection circuit 610 further detects an electrical impedance between at least one pair of the ECG electrodes 606. For example, the detection circuit 610 includes, or otherwise controls, a power source that applies a known voltage (or current) across a pair of the ECG electrodes 606 and detects a resultant current (or voltage) between the pair of the ECG electrodes 606. The impedance is generated based on the applied signal (voltage or current) and the resultant signal (current or voltage). In various cases, the impedance corresponds to respiration of the individual 608, chest compressions performed on the individual 608, and other physiological states of the individual 608. In various examples, the detection circuit 610 includes one or more analog filters configured to filter noise and/or artifact from the resultant signal. The detection circuit 610 generates a digital signal indicative of the impedance using an ADC. This digital signal can be referred to as an "impedance signal" or an "impedance."

The detection circuit 610 provides the ECG signal and/or the impedance signal one or more processors 612 in the external defibrillator 600. In some implementations, the processor(s) 612 includes a central processing unit (CPU), a graphics processing unit (GPU), both CPU and GPU, or other processing unit or component known in the art.

The processor(s) 612 is operably connected to memory 614. In various implementations, the memory 614 is volatile (such as random access memory (RAM)), non-volatile (such as read only memory (ROM), flash memory, etc.) or some combination of the two. The memory 614 stores instructions that, when executed by the processor(s) 612, causes the processor(s) 612 to perform various operations. In various examples, the memory 614 stores methods, threads, processes, applications, objects, modules, any other sort of executable instruction, or a combination thereof. In some cases, the memory 614 stores files, databases, or a combination thereof. In some examples, the memory 614 includes, but is not limited to, RAM, ROM, electrically erasable programmable read-only memory (EEPROM), flash memory, or any other memory technology. In some examples, the memory 614 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 612 and/or the external defibrillator 600. In some cases, the memory 614 at least temporarily stores the ECG signal and/or the impedance signal.

In various examples, the memory 614 includes a detector 616, which causes the processor(s) 612 to determine, based on the ECG signal and/or the impedance signal, whether the individual 608 is exhibiting a particular heart rhythm. For instance, the processor(s) 612 determines whether the individual 608 is experiencing a shockable rhythm that is treatable by defibrillation. Examples of shockable rhythms include ventricular fibrillation (VF) and ventricular tachycardia (VT). In some examples, the processor(s) 612 determines whether any of a variety of different rhythms (e.g., asystole, sinus rhythm, atrial fibrillation (AF), etc.) are present in the ECG signal.

The processor(s) 612 is operably connected to one or more input devices 618 and one or more output devices 620. Collectively, the input device(s) 618 and the output device(s) 620 function as an interface between a user and the defibrillator 600. The input device(s) 618 is configured to receive an input from a user and includes at least one of a keypad, a cursor control, a touch-sensitive display, a voice input device (e.g., a speaker), a haptic feedback device, or any combination thereof. The output device(s) 620 includes at least one of a display, a speaker, a haptic output device, a printer, or any combination thereof. In various examples, the processor(s) 612 causes a display among the input device(s) 618 to visually output a waveform of the ECG signal and/or the impedance signal. In some implementations, the input device(s) 618 includes one or more touch sensors, the output device(s) 620 includes a display screen, and the touch sensor(s) are integrated with the display screen. Thus, in some cases, the external defibrillator 600 includes a screen configured to visually output physiological parameters, such as the ECG signal and/or the impedance signal. In some cases, the screen is integrated with one or more touch sensors configured to receive user input signal(s).

In some implementations, the output device(s) 620 include a display that visually presents an enhanced user interface that guides an untrained user in operating the defibrillator 600. For example, the processor(s) 612 may execute instructions that cause the display to activate and deactivate various icons instructing the user. Icons corresponding to instructions that are irrelevant to a current step in operating the defibrillator 600 are deactivated, whereas icons corresponding to instructions that are relevant to the current step in operating the defibrillator 600 are activated. In some cases, deactivated icons are imperceptible to the user. Thus, the deactivated icons are prevented from distracting the user.

In some examples, the memory 614 includes an advisor 623, which, when executed by the processor(s) 612, causes the processor(s) 612 to generate advice and/or control the output device(s) 620 to output the advice to a user (e.g., a rescuer). In some examples, the processor(s) 612 provides, or causes the output device(s) 620 to provide, an instruction to perform cardiopulmonary resuscitation (CPR) on the individual 608. In some cases, the processor(s) 612 evaluates, based on the ECG signal, the impedance signal, or other physiological parameters, CPR being performed on the individual 608 and causes the output device(s) 620 to provide feedback about the CPR in the instruction. According to some examples, the processor(s) 612, upon identifying that a shockable rhythm is present in the ECG signal, causes the output device(s) 620 to output an instruction and/or recommendation to administer a defibrillation shock to the individual 608.

The memory 614 also includes an initiator 624 which, when executed by the processor(s) 612, causes the processor(s) 612 to control other elements of the external defibrillator 600 in order to administer a defibrillation shock to the individual 608. In some examples, the processor(s) 612 executing the initiator 624 selectively causes the administration of the defibrillation shock based on determining that the individual 608 is exhibiting the shockable rhythm and/or based on an input from a user (received, e.g., by the input device(s) 618. In some cases, the processor(s) 612 causes the defibrillation shock to be output at a particular time, which is determined by the processor(s) 612 based on the ECG signal and/or the impedance signal.

The processor(s) 612 is operably connected to a charging circuit 622 and a discharge circuit 625. In various implementations, the charging circuit 622 includes a power source 626, one or more charging switches 628, and one or more capacitors 630. The power source 626 includes, for instance, a battery. The processor(s) 612 initiates a defibrillation shock by causing the power source 626 to charge at least one capacitor among the capacitor(s) 630. For example, the processor(s) 612 activates at least one of the charging switch(es) 628 in the charging circuit 622 to complete a first circuit connecting the power source 626 and the capacitor to be charged. Then, the processor(s) 612 causes the discharge circuit 625 to discharge energy stored in the charged capacitor across a pair of defibrillation electrodes 634, which are in contact with the individual 608. For example, the processor(s) 612 deactivates the charging switch(es) 628 completing the first circuit between the capacitor(s) 630 and the power source 626, and activates one or more discharge switches 632 completing a second circuit connecting the charged capacitor 630 and at least a portion of the individual 608 disposed between defibrillation electrodes 634.

The energy is discharged from the defibrillation electrodes 634 in the form of a defibrillation shock. For example, the defibrillation electrodes 634 are connected to the skin of the individual 608 and located at positions on different sides of the heart of the individual 608, such that the defibrillation shock is applied across the heart of the individual 608. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 632 are controlled by the processor(s) 612, for example. In various implementations, the defibrillation electrodes 634 are connected to defibrillation leads 636. The defibrillation leads 636 are connected to a defibrillation port 638, in implementations. According to various examples, the defibrillation leads 636 are removable from the defibrillation port 638. For example, the defibrillation leads 636 are plugged into the defibrillation port 638.

In various implementations, the processor(s) 612 is operably connected to one or more transceivers 640 that transmit and/or receive data over one or more communication networks 642. For example, the transceiver(s) 640 includes a network interface card (NIC), a network adapter, a local area network (LAN) adapter, or a physical, virtual, or logical address to connect to the various external devices and/or systems. In various examples, the transceiver(s) 640 includes any sort of wireless transceivers capable of engaging in wireless communication (e.g., radio frequency (RF) communication). For example, the communication network(s) 642 includes one or more wireless networks that include a 3^{rd} Generation Partnership Project (3GPP) network, such as a Long Term Evolution (LTE) radio access network (RAN) (e.g., over one or more LE bands), a New Radio (NR) RAN (e.g., over one or more NR bands), or a combination thereof. In some cases, the transceiver(s) 640 includes other wireless modems, such as a modem for engaging in WI-FI^{®}, WIGIG^{®}, WIMAX^{®}, BLUETOOTH^{®}, or infrared communication over the communication network(s) 642.

The defibrillator 600 is configured to transmit and/or receive data (e.g., ECG data, impedance data, data indicative of one or more detected heart rhythms of the individual 608, data indicative of one or more defibrillation shocks administered to the individual 608, etc.) with one or more external devices 644 via the communication network(s) 642. The external devices 644 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 642. In some examples, the external device(s) 644 is located remotely from the defibrillator 600, such as at a remote clinical environment (e.g., a hospital). According to various implementations, the processor(s) 612 causes the transceiver(s) 640 to transmit data to the external device(s) 644. In some cases, the transceiver(s) 640 receives data from the external device(s) 644 and the transceiver(s) 640 provide the received data to the processor(s) 612 for further analysis.

In various implementations, the external defibrillator 600 also includes a housing 646 that at least partially encloses other elements of the external defibrillator 600. For example, the housing 646 encloses the detection circuit 610, the processor(s) 612, the memory 614, the charging circuit 622, the transceiver(s) 640, or any combination thereof. In some cases, the input device(s) 618 and output device(s) 620 extend from an interior space at least partially surrounded by the housing 646 through a wall of the housing 646. In various examples, the housing 646 acts as a barrier to moisture, electrical interference, and/or dust, thereby protecting various components in the external defibrillator 600 from damage.

In some implementations, the external defibrillator 600 is an automated external defibrillator (AED) operated by an untrained user (e.g., a bystander, layperson, etc.) and can be operated in an automatic mode. In automatic mode, the processor(s) 612 automatically identifies a rhythm in the ECG signal, makes a decision whether to administer a defibrillation shock, charges the capacitor(s) 630, discharges the capacitor(s) 630, or any combination thereof. In some cases, the processor(s) 612 controls the output device(s) 620 to output (e.g., display) a simplified user interface to the untrained user. For example, the processor(s) 612 refrains from causing the output device(s) 620 to display a waveform of the ECG signal and/or the impedance signal to the untrained user, in order to simplify operation of the external defibrillator 600.

In some examples, the external defibrillator 600 is a monitor-defibrillator utilized by a trained user (e.g., a clinician, an emergency responder, etc.) and can be operated in a manual mode or the automatic mode. When the external defibrillator 600 operates in manual mode, the processor(s) 612 cause the output device(s) 620 to display a variety of information that may be relevant to the trained user, such as waveforms indicating the ECG data and/or impedance data, notifications about detected heart rhythms, and the like.

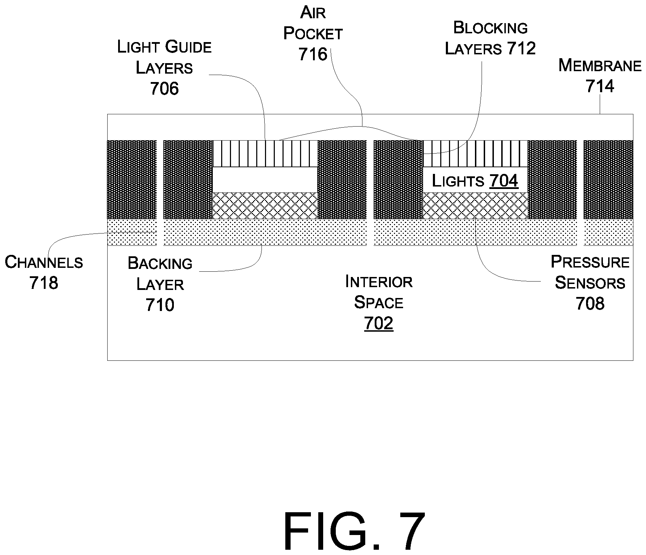

FIG. 7 illustrates an example of a screen of a medical device, which can implement various enhanced user interfaces described herein. Specifically, FIG. 7 illustrates a cross-sectional view of an example of a touch screen that can be utilized with various implementations described herein. Various elements described with respect to FIG. 7 can include one or more materials, such as metals, polymers (e.g., polyamide, polyethylene terephthalate (PET), etc.), ceramics, or the like.

The screen is disposed between an interior space 702 of the medical device and an exterior space outside of the medical device. The interior space 702, for instance, includes various circuits, sensors, and other functional elements of the medical device. In various cases, the interior space 702 is substantially fluid-tight. That is, the interior space 702 is sealed from the exterior space outside of the medical device by the screen and other elements of a housing of the medical device.

The screen includes various layers that enable the screen to output visual signals to a user, and to detect input signals from the user. For instance, the screen includes various lights 704 configured to output light signals through light guide layers 706. The light guide layers 706 are transparent and are configured to transmit the light from the lights 704. The light guide layers 706 include one or more transparent materials. Optionally, the light guide layers 706 are colored and/or shaped in a manner consistent with icons displayed by the medical device. The screen additionally includes pressure sensors 708 disposed between the lights 704 and the interior space 702. A backing layer 710 is disposed between the pressure sensors 708 and the interior space 702. For instance, the backing layer 710 provides structural support for the lights 704 and the pressure sensors 708.

In various implementations, the screen includes multiple lights 704 that respectively illuminate different icons of the device. To prevent the lights 704 from inadvertently illuminating neighboring light guide layers 706 associated with other icons, various blocking layers 712 are disposed between the lights 704 and light guide layers 706. In the implementation illustrated in FIG. 7, the blocking layers 712 are additionally disposed between the pressure sensors 708, but implementations are not so limited. The blocking layers 712 include one or more opaque materials that prevent light transmission.

According to various cases, a membrane 714 is disposed on an outer surface of the medical device. The membrane 714 may be transparent, flexible, and configured to prevent fluids (e.g., gasses, liquids, etc.) and/or particulate matter from entering the interior space 702 of the medical device from the exterior space. However, during manufacturing, it may be difficult to impossible to prevent the formation of an air pocket 716 from forming between the membrane 714 and the rest of the elements of the screen. The air pocket 716 may cause distortion of the light signals output by the light guide layers 706 through the membrane 714 and to a user. Moreover, the air pocket 716 may interfere with the detection of a touch signal from a user by the pressure sensors 708.

These and other complications of the air pocket 716 can be addressed by venting the air pocket 716 into the interior space. In various implementations, channels 718 extend through elements of the screen that separate the membrane 714 from the interior space 702. For example, the channels 718 may extend through the backing layer 710 and/or the blocking layers 712. Due to the presence of the channels 718, air within the air pocket 716 may flow into the interior space 702, such as when the membrane 714 is applied with a pressure (e.g., a touch signal). Accordingly, the impact of the air pocket 716 on the function of the screen and the medical device can be reduced.

### EXAMPLE CLAUSES

1. An automated external defibrillator (AED), including: a detection circuit configured to detect an electrical signal indicative of an electrocardiogram (ECG) of a subject, the electrical signal being received by pads configured to be adhered to the chest of the subject and that are electrically connected to the detection circuit; a treatment circuit configured to output an electrical shock to the pads; a screen including: a background or a foreground including a black color; a pressure sensor configured to detect a touch signal from a user; a shock icon corresponding to a shock command, the shock icon overlaid on the pressure sensor; a cardiopulmonary resuscitation (CPR) icon corresponding to an instruction to administer chest compressions; and a hands-off icon corresponding to an instruction to refrain from administering chest compressions; and a processor configured to: cause the screen to illuminate the shock icon, to illuminate the hands-off icon, and to blacken the CPR icon; in response to causing the screen to illuminate the shock icon, to illuminate the hands-off icon, and to blacken the CPR icon, determine that the ECG is indicative of ventricular fibrillation (VF) by analyzing the ECG; in response to determining that the ECG is indicative of VF, cause the screen to illuminate the shock icon, to blacken the hands-off icon, and to blacken the CPR icon; in response to causing the screen to illuminate the shock icon, to blacken the hands-off icon, and to blacken the CPR icon, determine that the pressure sensor has detected the touch signal; in response to determining that the pressure sensor has detected the touch signal, cause the treatment circuit to output the electrical shock to the pads; and in response to causing the treatment circuit to output the electrical shock to the pads, cause the screen to blacken the shock icon, to blacken the hands-off icon, and to illuminate the CPR icon.
2. The AED of clause 1, the pressure sensor being a first pressure sensor, the touch signal being a first touch signal, wherein the screen further includes: a second pressure sensor; a power icon overlaid on the second pressure sensor, and wherein the processor is further configured to: determine that the second pressure sensor has detected a second touch signal; and in response to determining that the second pressure sensor has detected the second touch signal, cause the touchscreen to illuminate the power icon, to blacken the shock icon, to blacken the CPR icon, and to blacken the hands-off icon.
3. The AED of clause 2, wherein the power icon is disposed between the CPR icon and the hands-off icon.
4. A medical device, including: a sensor configured to detect a physiological parameter of a subject; a screen including: a pressure sensor configured to detect a touch signal from a user; a first treatment icon corresponding to a command to administer a first treatment, the first treatment icon being icon overlaid on the pressure sensor; a second treatment icon corresponding to an instruction to administer a second treatment; and a pause icon corresponding to an instruction to refrain from administering the second treatment; and a processor configured to: cause the screen to illuminate the first treatment icon, to illuminate the pause icon, and to de-illuminate the second treatment icon; determine that the physiological parameter is indicative of a condition by analyzing the physiological parameter; in response to determining that the physiological parameter is indicative of the condition, cause the screen to illuminate the first treatment icon, to de-illuminate the pause icon, and to de-illuminate the second treatment icon; in response to causing the screen to the first treatment icon, to de-illuminate the pause icon, and to de-illuminate the second treatment icon, determine that the pressure sensor has detected the touch signal; in response to determining that the pressure sensor has detected the touch signal, cause the first treatment to be administered to the subject.
5. The medical device of clause 4, wherein de-illuminating the pause icon causes a color of the pause icon to match a color of a background or a foreground of the screen, and wherein de-illuminating the second treatment icon causes a color of the second treatment icon to match the color of the background or the foreground of the screen.
6. The medical device of clause 4 or 5, further including: a treatment circuit configured to administer the first treatment, wherein the first treatment includes an electrical shock or pacing pulses.
7. The medical device of any of clauses 4 to 6, wherein the second treatment includes chest compressions.
8. The medical device of any of clauses 4 to 7, wherein the condition includes VF, pulseless ventricular tachycardia (VT), or bradycardia.
9. The medical device of any of clauses 4 to 8, further including: the pressure sensor being a first pressure sensor, wherein the screen further includes: a second pressure sensor; and a power icon overlaid on the second pressure sensor, and wherein the processor is further configured to, in response to determining that the physiological parameter is indicative of the condition, cause the screen to de-illuminate the power icon.
10. The medical device of clause 9, the touch signal being a first touch signal, wherein the medical device is configured to power on in response to the first pressure sensor or the second pressure sensor detecting a second touch signal.
11. The medical device of any of clauses 4 to 10, wherein causing the screen to illuminate the first treatment icon, to de-illuminate the pause icon, and to de-illuminate the second treatment icon includes exclusively illuminating the first treatment icon and hiding other icons including the pause icon and the second treatment icon.
12. The medical device of clause 11, wherein the other icons further include a setup icon corresponding to an instruction to connect the sensor to the subject, a language selection icon, a pediatric mode icon, and a power icon.
13. A method, including: illuminating, at a screen, a first treatment icon and a pause icon, the first treatment icon corresponding to a command to administer a first treatment to a subject, the pause icon corresponding to an instruction to refrain from administering a second treatment to the subject; de-illuminating, at the screen, a second treatment icon corresponding to an instruction to administer the second treatment to the subject; determining that a physiological parameter of the subject is indicative of a condition by analyzing the physiological parameter; in response to determining that the physiological parameter of the subject is indicative of the condition: illuminating, at the screen, the first treatment icon; and de-illuminating the pause icon and the second treatment icon; detecting, by a pressure sensor overlaid by the first treatment icon, a touch signal; in response to detecting the touch signal, cause administration of the first treatment to the subject.
14. The method of clause 13, wherein de-illuminating the pause icon causes a color of the pause icon to match a color of a background or a foreground of the screen, and wherein de-illuminating the second treatment icon causes a color of the second treatment icon to match the color of the background or the foreground of the screen.
15. The method of clause 13 or 14, wherein the first treatment includes an electrical shock or pacing pulses.
16. The method of any of clauses 13 to 15, wherein the second treatment includes chest compressions or assisted ventilation.
17. The method of any of clauses 13 to 16, wherein the condition includes VF, pulseless ventricular tachycardia (VT), or bradycardia.
18. The method of clause 17, the touch signal being a first touch signal, the method further including: detecting, by the pressure sensor overlaid by the first treatment icon, a second touch signal; and in response to detecting the second touch signal, powering on a medical device including the screen.
19. The method of any of clauses 13 to 18, wherein illuminating the first treatment icon and de-illuminating the pause icon and the second treatment icon includes exclusively illuminating the first treatment icon and hiding other icons including the pause icon and the second treatment icon.
20. A method, including: illuminating, at a screen, a first treatment icon and a pause icon, the first treatment icon corresponding to a command to administer a first treatment to a subject, the pause icon corresponding to an instruction to refrain from administering a second treatment to the subject; de-illuminating, at the screen, a second treatment icon corresponding to an instruction to administer the second treatment to the subject; determining that a physiological parameter of the subject is indicative of a condition by analyzing the physiological parameter; in response to determining that the physiological parameter of the subject is indicative of the condition: illuminating, at the screen, the first treatment icon; and de-illuminating the pause icon and the second treatment icon; determining that a predetermined time period has expired without a pressure sensor overlaid by the first treatment icon detecting a touch signal; and in response to determining that the predetermined time period has expired without the pressure sensor overlaid by the first treatment icon detecting the touch signal, cause administration of the first treatment to the subject.
21. An automated external defibrillator (AED), including: a detection circuit configured to detect an electrical signal indicative of an electrocardiogram (ECG) of a subject, the electrical signal being received by pads configured to be adhered to the chest of the subject and that are electrically connected to the detection circuit; a treatment circuit configured to output an electrical shock to the pads; a screen including: a background or a foreground including a black color; a first pressure sensor; a shock icon corresponding to a shock command, the shock icon being overlaid on the first pressure sensor; a second pressure sensor; a power icon overlaid on the second pressure sensor; an instruction icon corresponding to an instruction to place the pads on the chest of the subject; a processor configured to: determine that the first pressure sensor received a first touch signal from a user; in response to determining that the first pressure sensor received the touch signal from the user, cause the screen to illuminate the shock icon and to illuminate the instruction icon; determine that the ECG is indicative of ventricular fibrillation (VF); determine that the first pressure sensor has received a second touch signal from a user; and in response to determining that the ECG is indicative of VF and that the first pressure sensor has received the second touch signal from the user, cause the treatment circuit to output the electrical shock to the pads.
22. The AED of clause 21, wherein the instruction icon indicates a position to apply the pads to the chest of the subject.
23. The AED of clause 21 or 22, wherein the processor is further configured to: in response to determining that the ECG is indicative of VF, cause the screen to illuminate the shock icon and to blacken other icons, the other icons including the instruction icon.
24. A medical device, including: a physiological parameter sensor configured to detect a physiological parameter of a subject; a treatment circuit configured to administer a treatment to the subject; a screen including: a first pressure sensor; a treatment icon corresponding to a command to administer the treatment to the subject, the treatment icon being overlaid on the first pressure sensor; a second pressure sensor; a power icon overlaid on the second pressure sensor; an instruction icon corresponding to an instruction to connect the physiological parameter sensor to the subject; a processor configured to: cause the screen to illuminate the treatment icon, to illuminate the power icon, and to de-illuminate the instruction icon; determine that the first pressure sensor received a touch signal from a user; in response to determining that the first pressure sensor received the touch signal from the user, cause the screen to illuminate the treatment icon and to illuminate the instruction icon.
25. The medical device of clause 24, wherein the treatment includes an electrical shock or pacing pulses.
26. The medical device of clause 24 or 25, wherein the physiological parameter sensor includes a monitoring circuit configured to detect, from electrodes configured to be adhered to the chest of the subject, an electrical signal indicative of an electrocardiogram (ECG) of the subject.
27. The medical device of any of clauses 24 to 26, wherein the processor is configured to power on the medical device in response to determining that the first pressure sensor received the touch signal from the user.
28. The medical device of clause 27, further including: a power source including a battery; and a switch configured to selectively connect the battery to the physiological parameter sensor, wherein the processor is configured to power on the medical device by causing the switch to selectively connect the battery to the physiological parameter sensor.
29. The medical device of any of clauses 24 to 28, wherein the treatment icon includes a backlit shape.
30. The medical device of clause 29, wherein the treatment icon overlaps a center of the screen.
31. The medical device of any of clauses 24 to 30, the treatment being a first treatment, the screen further including: a second treatment icon corresponding to an instruction, to a user, to manually administer a second treatment to the subject.
32. The medical device of clause 31, wherein the second treatment includes chest compressions or assisted ventilation.
33. A method, including: illuminating, at a screen, a treatment icon and a power icon, the treatment icon corresponding to a command to administer a treatment to a subject, the power icon corresponding to powering on a medical device including the screen; de-illuminating, at the screen, an instruction icon corresponding to an instruction to connect the medical device to a subject; detecting, by a pressure sensor overlaid with the treatment icon, a touch signal from a user; and in response to detecting the touch signal from the user: illuminating, at the screen, the treatment icon and the instruction icon.
34. The method of clause 33, wherein the treatment includes an electrical shock or pacing pulses.
35. The method of clause 33 or 34, wherein the medical device includes a monitoring circuit configured to detect, from electrodes configured to be adhered to the chest of the subject, an electrical signal indicative of an electrocardiogram (ECG) of the subject.
36. The method of any of clauses 33 to 35, further including: in response to detecting the touch signal from the user, powering on the medical device.
37. The method of clause 36, wherein powering on the medical device includes connecting a battery to a physiological parameter sensor in the medical device.
38. The method of any of clauses 33 to 37, wherein the treatment icon includes a first backlit shape, the power icon includes a second backlit shape, and the instruction icon includes a third backlit shape.
39. The method of any of clauses 33 to 38, the treatment being a first treatment, the method further including: illuminating, by the screen, a second treatment icon instructing a user to manually administer a second treatment to the subject.
40. The method of clause 39, wherein the second treatment includes chest compressions or assisted ventilation.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of' excludes any element, step, ingredient or component not specified. The transition phrase "consisting essentially of' limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11 % of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1 % of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, comprising:
a sensor configured to detect a physiological parameter of a subject;
a screen comprising:
a pressure sensor configured to detect a touch signal from a user;
a first treatment icon corresponding to a command to administer a first treatment, the first treatment icon being overlaid on the pressure sensor;
a second treatment icon corresponding to an instruction to administer a second treatment; and
a pause icon corresponding to an instruction to refrain from administering the second treatment; and
a processor configured to:
cause the screen to illuminate the first treatment icon, to illuminate the pause icon, and to de-illuminate the second treatment icon;
determine that the physiological parameter is indicative of a condition by analyzing the physiological parameter;
in response to determining that the physiological parameter is indicative of the condition, cause the screen to illuminate the first treatment icon, to de-illuminate the pause icon, and to de-illuminate the second treatment icon;
in response to causing the screen to illuminate the first treatment icon, to de-illuminate the pause icon, and to de-illuminate the second treatment icon, determine that the pressure sensor has detected the touch signal; and
in response to determining that the pressure sensor has detected the touch signal, cause the first treatment to be administered to the subject.

2. The medical device of claim 1, wherein de-illuminating the pause icon causes a color of the pause icon to match a color of a background or a foreground of the screen, and/or
wherein de-illuminating the second treatment icon causes a color of the second treatment icon to match the color of the background or the foreground of the screen.

3. The medical device of claim 1 or 2, further comprising:
a treatment circuit configured to administer the first treatment,
wherein the first treatment comprises an electrical shock or pacing pulses.

4. The medical device of claim 1, 2 or 3, wherein the second treatment comprises chest compressions.

5. The medical device of claims 1-4, wherein the medical device is an automated external defibrillator (AED),
wherein the sensor comprises a detection circuit configured to detect an electrical signal indicative of an electrocardiogram (ECG) of a subject, the electrical signal being received by pads configured to be adhered to the chest of the subject and that are electrically connected to the detection circuit;
wherein the treatment circuit is configured to output an electrical shock to the pads;
wherein the screen comprises:
a background or a foreground comprising a black color;
wherein the first treatment icon is a shock icon corresponding to a shock command, the shock icon overlaid on the pressure sensor;
wherein the second treatment icon is a cardiopulmonary resuscitation (CPR) icon corresponding to an instruction to administer chest compressions; and
wherein the pause icon is a hands-off icon corresponding to an instruction to refrain from administering chest compressions; and
wherein the processor is configured to:
cause the screen to illuminate the shock icon, to illuminate the hands-off icon, and to blacken the CPR icon;
in response to causing the screen to illuminate the shock icon, to illuminate the hands-off icon, and to blacken the CPR icon, determine that the ECG is indicative of ventricular fibrillation (VF) by analyzing the ECG;
in response to determining that the ECG is indicative of VF, cause the screen to illuminate the shock icon, to blacken the hands-off icon, and to blacken the CPR icon;
in response to causing the screen to illuminate the shock icon, to blacken the hands-off icon, and to blacken the CPR icon, determine that the pressure sensor has detected the touch signal;
in response to determining that the pressure sensor has detected the touch signal, cause the treatment circuit to output the electrical shock to the pads; and
in response to causing the treatment circuit to output the electrical shock to the pads, cause the screen to blacken the shock icon, to blacken the hands-off icon, and to illuminate the CPR icon.

6. The medical device of any of claims 1-5, wherein the condition comprises VF, pulseless ventricular tachycardia (VT), or bradycardia.

7. The medical device of any of claims 1-6, further comprising:
the pressure sensor being a first pressure sensor, wherein the screen further comprises:
a second pressure sensor; and
a power icon overlaid on the second pressure sensor, and
wherein the processor is further configured to, in response to determining that the physiological parameter is indicative of the condition, cause the screen to de-illuminate the power icon.

8. The medical device of claim 7, as far as dependent from claim 5,
wherein the processor is further configured to:
determine that the second pressure sensor has detected a second touch signal; and
in response to determining that the second pressure sensor has detected the second touch signal, cause the screen to illuminate the power icon, to blacken the shock icon, to blacken the CPR icon, and to blacken the hands-off icon.

9. The medical device of claim 8, wherein the power icon is disposed between the CPR icon and the hands-off icon.

10. The medical device of any of claims 7-9, the touch signal being a first touch signal, wherein the medical device is configured to power on in response to the first pressure sensor or the second pressure sensor detecting a second touch signal.

11. The medical device of any of claims 1-10, wherein causing the screen to illuminate the first treatment icon, to de-illuminate the pause icon, and to de-illuminate the second treatment icon comprises exclusively illuminating the first treatment icon and hiding other icons comprising the pause icon and the second treatment icon, wherein the other icons optionally further comprise a setup icon corresponding to an instruction to connect the sensor to the subject, a language selection icon, a pediatric mode icon, and a power icon.

12. A method, comprising:
illuminating, at a screen, a first treatment icon and a pause icon, the first treatment icon corresponding to a command to administer a first treatment to a subject, the pause icon corresponding to an instruction to refrain from administering a second treatment to the subject;
de-illuminating, at the screen, a second treatment icon corresponding to an instruction to administer the second treatment to the subject;
determining that a physiological parameter of the subject is indicative of a condition by analyzing the physiological parameter;
in response to determining that the physiological parameter of the subject is indicative of the condition:
illuminating, at the screen, the first treatment icon; and
de-illuminating the pause icon and the second treatment icon;
detecting, by a pressure sensor overlaid by the first treatment icon, a touch signal;
in response to detecting the touch signal, generating a command to cause administration of the first treatment to the subject.

13. The method of claim 12, wherein de-illuminating the pause icon causes a color of the pause icon to match a color of a background or a foreground of the screen, and/or
wherein de-illuminating the second treatment icon causes a color of the second treatment icon to match the color of the background or the foreground of the screen.

14. The method of claim 12 or 13, wherein the first treatment comprises an electrical shock or pacing pulses, and/or the second treatment comprises chest compressions or assisted ventilation.

15. The method of claim 12, 13 or 14, wherein the condition comprises VF, pulseless ventricular tachycardia (VT), or bradycardia; optionally the touch signal being a first touch signal and the method further comprising:
detecting, by the pressure sensor overlaid by the first treatment icon, a second touch signal; and
in response to detecting the second touch signal, powering on a medical device comprising the screen.
